# EUROPEAN PATENT APPLICATION

(11) **EP 3 508 206 A1**
(43) Date of publication of application: **10.07.2019**
(21) Application number: 16914377.3
(22) Date of filing: 01.09.2016
(51) Int. Cl.: A61K 35/14, A61K 35/16, A61K 35/18, A61K 38/38, A61K 38/42, A61K 39/395, A61K 47/14, A23L 33/00, A61P 7/06, A61P 31/00, A61P 35/00, A61P 37/00

(54) **FOOD FACTOR-BASED FORMULATION, PRODUCTS PRODUCED USING SAID FORMULATION AND METHODS FOR PRODUCING SAME**

(71) Applicant: Bebeachibuli, Romeo, 13560-230 São Carlos - SP (BR)
(72) Inventor: Bebeachibuli, Romeo, 13560-230 São Carlos - SP (BR)
(74) Representative: Urizar Anasagasti, Jesus Maria
(86) International application number: PCT/BR2016/050215
(87) International publication number: WO 2018/039754

(57) **Abstract**

The present abstract relates to an invention patent application for a food factor-based formulation, products produced therewith and method for producing same, pertaining to the field of products of food and medical-pharmaceutical use, developed for numerous treatments for humans, particularly products which can be recognized or registered by the immune system as an antigen; said formulation comprising a blood or blood fraction derivative combined with phospholipids, preferably phosphatidylcholine in an aqueous or serological solution.

## Description

The present specification relates to an application for patent for a formulation based on dietary factors, products made therewith and the process of obtaining belonging to the field of products for medical, pharmacological and nutritional use in generally, developed for many ancillary treatments for human diseases, particularly immune system, anemia and others that can be registered or recognized by the immune system as the antigen. The product is a dietary supplement, strengthening, and general activator for healthy or weak individuals, state of malnutrition and prophylactic use, trivializing the severity of new diseases that can be acquired in the near future.

### STATE OF THE ART

It is known by conventional medicine the difficulties encountered in increasing the body's defense in order to combat diseases linked to the defense of the body, insufficiency of the immune system and its malfunction. Moreover, we observed this obstacle also in the treatment of various types of anemia, both those with genetic defect such as thalassemia - hereditary condition known as anemia of the Mediterranean.

In order to help in the immune response "conventional medicine", after a critical evaluation, uses in patients, among other methods, gama globulin specific proteins isolated from humans, horse antivenom serum and antiserum injected intramuscularly, subcutaneously or intravenously. These techniques help the patient. However, after repeated application, can cause shock or clotting which may be followed by embolism or dangerous allergic reactions, detrimental to the body's immune response in the formation of antibodies, because they are proteins "foreign to the organism" and therefore must be eliminated. This implies to say that this whole process complicates and overwhelms the immune defense of the person.

In the beginning, gamma globulin was freely sold in pharmacies, but currently is limited to hospital use, but the problem persists.

The conventional treatments of various types of anemia are based on the replacement of the various components required for the production of red blood cells, such as ferrous sulphate, folic acid, vitamin B12, vitamin C, etc., which are mostly dietary factors. These types of treatments are effective, but are of long term which, in case of some substances, such as the case of iron, may typically cause allergies, stomach ulcers, and becomes 'heavy' to the stomach and its intravenous injectable form can affect the lining of the internal veins and the remainder of it is deposited in the body mainly affecting liver function. Therefore are treatments that work only in anemia caused by lack of the aforementioned components.

The anemia caused by genetic disorders is generally treated the same way but without effectiveness due to lack of necessary genes, using also, where appropriate, blood transfusion. The effect causes the formation of antibodies against the foreign blood and other complications in the body. The most modern treatments of anemia caused by genetic defects are made by implants of stem cells in the patients.

### OBJECTIVES OF THE INVENTION

The main objective of the present invention is to provide mediation, particularly as adjuvants for the treatment of immune system, anemia and others of humans, among others.

Another objective is to provide products with nutritional function, to help recover the natural physiological state of the body and assist in treatment of humans.

Another objective is to provide a process for obtaining the formulation from which the products are obtained.

### BRIEF DESCRIPTION OF THE INVENTION

The inventor's findings, from which the formulation was developed and products that are applied for the present invention are briefly described below. Thus, in view of the prior art and its problems and limitations, above alluded to, and to meet the related objectives were developed based formulation of dietary factors, products obtained from the use thereof and the process of obtaining the formulation, objects present invention, said formulation based on dietary factors, including blood fractions derived from blood or combined with phospholipids or their cell membrane fractions natural or synthetic, preferably, emulsion or bead phosphatidylcholine, type sold freely on the world market.

Experiments conducted found that this formulation and the products obtained from it are great natural food factors which help in the vital functions in the prevention of diseases in the body, in the recovery of patients and aid in the treatment of cancer and other human diseases which may be registered or recognized by the immune system as the antigen.

A positive effect, particularly obtained with the formulation and products and objects of the present invention is the use against thalassemia - Mediterranean anemia. Other positive effects and observations made in patients with certain weaknesses because of different diseases with mild to moderate case of deformation of the red blood cells attested by findings in laboratory tests and - similar to thalassemia - which disappeared after the recovery from the diseases of the body. Conclusions: Certain diseases can deform red blood during body weakness. This deformation is reversible upon recovery of the patient, with or without the use of products object of the present invention. The product, object of the present invention can only accelerate the recovery of these patients.

### DETAILED DESCRIPTION OF THE INVENTION

The present invention relates to a food factor-based formulation comprising mainly: derivatives from blood or blood fractions combined with cell membrane phospholipids or their natural or synthetic fractions, preferably phosphatidylcholine in solution; said invention further comprises the use of alluded formulation to obtain numerous efficient products for the treatment of human health; said products comprising alluded formulation and eventually adjuvants pharmaceutically and / or nutritionally acceptable.

### BACKGROUND OF THE INVENTION

The formulation development and products obtained by its use were made from the following observations:
1. The gamma globulin present in the pharmaceutical market, isolated from the blood serum of homologous origin to be applied intravenously as indicated, should be combined - uploaded and / or mixed- with biological stabilizer such as phospholipids of the cell membrane or its natural or synthetic fractions of preferably phosphatidylcholine, in physiologic solution or not, to lower its side effects.
   In addition, therefore, the present formulation based on dietary factors may be employed to prepare a product comprising an biological stabilizer to be combined with gamma globulin of isolated type of the blood serum of homologous origin, injectable; said biological stabilizer comprising: cell membrane phospholipids or their natural or synthetic fractions, preferably phosphatidylcholine in physiological solution or not in suitable proportions.
2. The gamma globulin present in the pharmaceutical market, isolated from the blood serum of homologous origin should be taken orally with or without stabilizer such as biological cell membrane phospholipids or their natural or synthetic fractions, preferably phosphatidylcholine in aqueous solution or other diluents or not, ensuring the desired effect of increasing the body's defense, without any negative side effects.
   Thus, the present formulation can be used to make a useful product to increase or enhance the defense capacity of the human body administered orally comprising: Isolated gamma globulin from blood serum of homologous origin, combined or not with biological stabilizer comprising cell membrane phospholipids or their natural or synthetic fractions, preferably phosphatidylcholine in aqueous solution or other diluents or not in suitable proportions.
3. The gamma globulin isolated from blood serum of heterologous origin of mammals, warm-blooded species, should be taken orally with or without stabilizer such as biological cell membrane phospholipids or their natural or synthetic fractions, preferably phosphatidylcholine in aqueous solution or other diluents or not, and pasteurized, assisting the desired effect of increasing the defense of the body without any negative side effects.
   In this case, the formulation can be used to make a product administered orally to increase the protection of the human body comprising gamma globulin isolated from blood serum of heterologous origin of mammals, combined or not with biological stabilizer such as phospholipids of the cellular membrane or its natural or synthetic fractions, preferably phosphatidylcholine in aqueous solution or other diluents or not pasteurized and, in suitable proportions.
4. The specific gamma globulin, antivenom against snake bites, antiserum to treat or neutralize poison of other animals and insects should be in an emergency, used at the beginning of treatment as indicated in conventional medicine. This treatment can be improved by using together with conventional injectable form, antiserum orally from the outset, with or without biological stabilizer such as phospholipids of the cell membrane or its natural or synthetic fractions, preferably phosphatidylcholine in aqueous solution or other diluents or not, and pasteurized, which has delayed effect because of the time needed to complete digestion. Thus the body can produce suitable antibodies, supplementing and replacing the need for use of more injections in severe cases.
   In this case, the formulation is used to prepare antiserum against the bite of poisonous animals for oral administration, said antiserum comprising specific gamma globulin against bites of venomous animals, combined or not with biological stabilizer such as phospholipids of the cell membrane or its natural fractions or synthetic preferably phosphatidylcholine in aqueous solution or other diluents or not, and pasteurized in suitable proportions.
5. The serum extracted from the blood of heterologous origin in mammals, with or without biological stabilizer such as phospholipids of the cell membrane or its natural or synthetic fractions, preferably phosphatidylcholine, dissolved in aqueous or other diluents or not, and pasteurized, helps to potentially increase the effectiveness of the immune system and improve the metabolism of the treated patients when used orally, without causing side effects or counter indications. It is evaluated as a nutritional factor.
   Thus, the formulation may be used to prepare oral nutritional product comprising serum extracted from of heterologous origin in mammals, combined or not with biological stabilizer comprising cell membrane phospholipids and their natural or synthetic fractions, preferably phosphatidylcholine, dissolved in aqueous or other diluents or not, and pasteurized in suitable proportions.
6. The combination of phospholipids of the cell membrane solution or its natural or synthetic fractions preferably phosphatidylcholine and blood serum of heterologous origin of mammals, with natural juices, provide the three necessary food components: fat, protein, carbohydrate ensuring an energizing effect and optimal nutrition more complete via oral and enteral.
   To address this, the formulation can be used to prepare oral and enteral energetic product comprising a combination of solution of phospholipids of the cell membrane or its natural or synthetic fractions preferably phosphatidylcholine; blood serum of heterologous origin of mammals; natural juices, in appropriate proportions.
7. The red blood cells taken from blood, from heterologous origin of mammals after separated, hydrolyzed, "diluted with water" or not, to release hemoglobin from their cells and isolate the aqueous solution of hemoglobin or not, said red blood cells so processed combined or not with a biological stabilizer comprising cell membrane phospholipids or their natural or synthetic fractions, preferably phosphatidylcholine in aqueous solution or other diluents or not pasteurized and taken in appropriate oral fractions can help heal various types of anemia including thalassemia - Mediterranean anemia - which can improve blood pathological status or normalize it, depending on the degree of the disease and to eliminate or reduce the need for blood transfusion, offering complete combination for the formation of hemoglobin, red blood cells and their needed enzymes.
   In addition, the formulation based on dietary factors, object of the present invention can be used in product preparation auxiliary counter anemia, administered orally, comprising red blood cells taken from blood, from heterologous origin in mammals hydrolyzed "diluted" or not and whether or not combined with emulsifier, protective and biological stabilizer comprising phospholipids the cell membrane, or their natural or synthetic fractions, preferably phosphatidylcholine in aqueous solution or other diluents or not pasteurized in suitable ratios as needed. This product proves to be particularly effective to assist in treatment of thalassemia - Mediterranean anemia.
8. After the sterile collection of blood of healthy mammals fractions thereof can be tested or not for the case of existence of contagious diseases that may be transmitted to humans, the receiver stabilized with phospholipids of the cell membrane or its natural or synthetic fractions sterilized, preferably phosphatidylcholine, dissolved in aqueous or other diluents or not, divided into portions of practical dosages for oral use and frozen as quickly as possible to avoid losing large quantities of its natural qualities because without being pasteurized they have more positive effects on their healing properties.
   In this case, the formulation may be employed to prepare frozen product, adjuvant and strengthener against different diseases of humans, for administration in oral doses comprising of blood product or fractions of blood of mammals, most effective against disease, stabilized with phospholipids of the cell membrane or its natural fractions or synthetic, preferably phosphatidylcholine, dissolved in aqueous or other diluents or not in suitable proportions.
9. The blood of mammals, hydrolyzed or not, with or without biological stabilizer such as phospholipids of the cell membrane or its natural or synthetic fractions, preferably phosphatidylcholine, solution, or other aqueous diluents or not, the product acquired pasteurized, helps to increase the effectiveness of the immune system, to treat anemia and improve the metabolism of the treated patients when taken orally without causing side effects or counter indications.
   Thus, the formulation may be used to prepare oral product, to improve the immune system, the metabolism and help treat anemia, comprising blood of mammals, hydrolyzed or not, with or without biological stabilizer such as phospholipids of the cell membrane or its natural or synthetic fractions, preferably phosphatidylcholine, solution, aqueous or other diluents or not, in suitable proportions.
10. The infusion of whole blood or solution of red blood cells isolated from human blood, with biological stabilizer such as phospholipids of the cell membrane or its natural or synthetic fractions preferably phosphatidylcholine diluted with saline reduces the side effects of treatment. It decreases the shock form clot or hyper coagulation in the receiver.
   In this case, the formulation is used to prepare auxiliary product for treatment of humans, administered by infusion, comprising whole blood or solution of red blood cells isolated from human blood, with biological stabilizer such as phospholipids cell membrane or its natural or synthetic fractions preferably phosphatidylcholine diluted with saline, in suitable proportions.
11. It was observed the effectiveness of serum or its gamma globulin with specific antibodies, "antiserum" to treat individually infectious diseases and other pathologies, isolated of the blood of the mammals previously vaccinated against the disease target, combined or not with biological stabilizer comprising phospholipids cell membrane or its natural or synthetic fractions, preferably phosphatidylcholine in aqueous solution or other diluents or not, pasteurized or not, and taken orally, to help treatments of the target disease caused by any toxins, bacteria, viruses, cancer or ringworm . By this method serious human diseases can be treated more efficiently, helping to produce specific antibodies.
   In addition, the invented formulation can be used to prepare product of fragments of antibody auxiliary for the production of specific antibodies, administered orally, comprising serum isolated or its gamma globulin isolated from blood of warm-blooded mammals animals previously vaccinated against target disease or not combined with a biological stabilizer comprising cell membrane phospholipids or their natural or synthetic fractions, preferably phosphatidylcholine in aqueous solution or other diluents or not, pasteurized or not, in suitable proportions. At the end of this description, there is a description relating specifically to cancer, more recent studies of treatment of this disease and the possible association of treatment to the formulation of the present invention.
12. These primed products: serum, red blood cell or hemoglobin, gamma globulin, whole blood combined with phospholipids of the cell membrane or its natural or synthetic fractions, preferably phosphatidylcholine or their solutions, when necessary, can be used via oral as nutritional strengthening of great value in the case of serious diseases and malnutrition.
   The treatment with fractionated blood provides basic fractions to assemble humoral and cellular defense and thus helps in the treatment of immune deficiencies diseases, autoimmune diseases, bacterial, viral, fungal, in the prophylaxis and treatment of cancer before during or after conventional methods, diseases considered incurable in the current conventional medicine, various types of anemias and diseases of defective non dominant genes that cause missing or malformation of substances in the body. The treatment aids or corrects the body's metabolism, helps in the treatment and cure of all diseases which can be registered by the body as antigen.
13. The type of blood used from mammals or their blood fractions can be of cows, for quantitative reasons, or other herbivore that contains essential substances for the human body. The serum of carnivorous animals is more adapted to humans, in relation to the production of antibodies or other substances necessary and essential that may lack in the herbivorous animals. The blood fractions used can also be combined of carnivores and herbivores of different species. In the case of oral use, the blood type or blood group does not matter.

The above alluded products may comprise, eventually, additionally, adjuvants pharmaceutically and/or nutritionally acceptable in suitable proportions.

In addition, it is known that lecithin is a phospholipid of the natural cell membrane completely metabolized, is not excreted from the body in the feces or urine, it is considered and ranked by the Food and Drug Administration in the United States as non-toxic edible substance, safe for the human body and its overdose is not known. In thhe European Union is recognized as a food additive.

The cell membrane phospholipids are present in the national or international market in dry granular form, emulsion or capsules. In pharmacies they are sold as lecithin, phosphatidylcholine, phosphatidylinositol, phosphatidylethanolamine and others of its fractions extracted from soy, eggs, cereals or the like. Lecithin forms the cell membranes of all living beings, humans, animals and plants. Lecithin is combined phosphatidylserine, phosphatidylethanolamine, phosphatidylcholine and sphingomyelin and other non-toxic fractions, but is generally understood as phosphatidylcholine. All are characterized as nutritional supplements and protectors, stabilizers, emulsifiers, to carry medicines, genes or vaccines, as well as being used in radiological diagnosis.

The whole blood, erythrocytes, gamma globulin are used as infusions or injections in conventional medicine. A protein solution in enteral or parenteral applications.

The process of preparing the formulation or product essentially comprises:
1 - The serum, the gamma globulin, hemoglobin, red blood cells or blood are prepared at low temperature, stabilized with phospholipids of the cell membrane or its natural or synthetic fractions, preferably phosphatidylcholine, fractionated blood solution and then dissolved in water in equal volumes or not for oral use. The phospholipids may be sterilized before being mixed with other components. Finally, the product is pasteurized or not, divided into portions that require in each unit at least 10 ml of blood fraction, frozen to be conserved, stored and inhibit the damage of unstable fragments such as enzymes, proteins or others. Freezing can break cells, polymerized proteins or other, but without harming the expected end product. The recipe accepts various concentrations of its components without losing the desired effect.
   The method therefore comprises the steps of:
   - Preparing of blood derivatives or blood fractions (gamma globulin, hemoglobin, blood serum, red blood cells or blood) at low temperature of 3 to 10 degree Celsius for their preparation and their manipulations using refrigerator, water bath or water with ice cubes which is a standard known process and during handling of fresh biological substances;
   - Stabilizing the blood derivatives or blood fractions (gamma globulin, hemoglobin, blood serum, red blood cells or blood), prepared at low temperature via phospholipids of the cell membrane, or their natural or synthetic fractions preferably phosphatidylcholine, in the proportion of 1 to 500 grams in one liter of blood fractionated solution
   - Dissolving blood derivatives or from blood fractions (gamma globulin, hemoglobin, blood serum, red blood cells or blood) stabilized, emulsified and protected with cell membrane phospholipids or their natural or synthetic fractions, preferably phosphatidylcholine in water in equal volumes or not;
   - Optionally pasteurizing the formulation obtained;
   - Divide into portions (doses) comprising at least 10 ml per serving of blood fraction; and
   - Freezing.
2. The required amount of solution is taken orally immediately after thawing, according to need for each individual or as physical state or medical indication. The thawed vials can be taken diluted in water, plain milk or chocolate milk, yogurt, or natural juice depending on the taste of the receptor. The maximum daily dosage should be calculated according to the proteins offered in the meals.
3- The liquid or aqueous solution of phospholipids and blood fractions, once ready for use, can be lyophilized for distribution. Moreover, this dry product may be encapsulated and the aqueous or gelatin solution of blood fractions can be stored in gelatin, soft or hard, divided in portions to be distributed to be taken orally.

The tables below are substantially a summary of the invention. These tables refer to the standard proportion of blood and its fractions with phospholipid and indications of the main steps of the process related to the type of administration, oral and injectable, according to the present invention.

**Table 1 = For oral use of isolated blood serum or gamma globulin**

| REF. | INGREDIENT/STEPS | Quantities/ Validity |
|---|---|---|
| 1 | Gamma globulin or blood serum solution | 1 liter |
| 2 | Sterilized phospholipids | 0 to 500 g |
| 3 | Mix until homogenous solution | - |
| 4 | Add water or not | 1 L |
| 5 | Mix until homogenous solution | - |
| 6 | Pasteurize the product or not | - |
| 7 | Split the product or not | 50 to 100 portions |
| 8 | Freeze the product | -7 °C to -25 °C, validity of 4 months |
| 9 | Freeze the product | - 40 °C, validity of 6 to 8 months |
| 10 | Freeze product in liquid carbon dioxide | Validity of 5 years |
| 11 | Freeze product in liquid nitrogen | Unlimited validity |
| 12 | Thaw the product in warm water to be taken immediately | - |

**Table 2 = For oral use of gamma globulin present in the pharmaceutical market**

| REF. | INGREDIENT/STEP | Quantities/ Validity |
|---|---|---|
| 1 | Sterilized phospholipid | 0 to 500 g |
| 2 | Gama globulin | 1 liter |
| 3 | Clean water or natural juice | 1 liter |
| 4 | Mix until homogenous solution | - |
| 5 | Split the product | 50 to 100 portions |
| 6 | Freeze portions according to desired validity | - |
| 7 | Thaw the product in warm water to be taken immediately | - |

**Table 3 = For oral use of hemoglobin from red blood cells or blood.**

| REF. | INGREDIENT/STEP | Quantities/ Validity |
|---|---|---|
| 1 | Red blood cells or blood | 1 Liter |
| 2 | Add sterile distilled water | 0.5 Liter |
| 3 | Centrifuge the product or not | - |
| 4 | Sterilized phospholipids | 0 to 500g |
| 5 | Mix until homogenous solution | - |
| 6 | Add water or other diluent or not | 0 to 0.5 liter |
| 7 | Split the product | 50 to 100 portions |
| 8 | Freeze portions according to desired validity | - |
| 9 | Thaw the product in warm water to be taken immediately | - |

**Table 4 = For injectable use of gamma globulin present in the pharmaceutical market**

| REF. | INGREDIENT/STEP | Quantities/ Validity |
|---|---|---|
| 1 | Sterilized phospholipids | 1 to 5 grams |
| 2 | Gammaglobulin present in the pharmaceutical market | 1 Liter |
| 3 | Mix until homogenous solution | - |
| 4 | Saline solution or not | 1 Liter |
| 5 | Mix components until homogenous solution | - |
| 6 | Split the product | 500 Portions |
| 7 | Freeze the fractions according to the desired validity | - |
| 8 | Thaw each dose of the product at the time of use | - |

**Table 5 - For injectable use; of whole blood; red blood cell solution**

| REF. | INGREDIENT/STEP | Quantities/ Validity |
|---|---|---|
| 1 | Phospholipids | 1 to 5 grams |
| 2 | Dissolve in saline solution | 2 to 10ml |
| 3 | Mix until homogenous solution | - |
| 4 | Whole blood or erythrocytes | 1 liter |
| 5 | Mix until homogenous solution | - |
| 6 | Store at proper temperature | 3 to 8 Celsius |

### Justifications, arguments and instructions for use

To support the ideas mentioned above, natural processes were observed, analyzed, compared, and evaluated added logical interpretations of scientific studies and results in medical practice as follows:
1. Nature is economic. Thus, the body does not digest proteins completely to their amino acids, but it uses functional fragments ready to be reassembled, according to the momentary needs present in the body. Unnecessary fragments, like pieces of a particular type of protein from other species or dispensable at that time, will be dismantled or not, and valued in their own metabolism.
2. The blood of animals and their used fractions are less dangerous than those of humans, because of the lack of specific germs for the humans. Treatment with blood fractions orally requires some time to get to your desired response. This depends on the time needed to complete the digestion that removes fractions of specific heterologous fractions of animals in the metabolism of the receptor; the remaining fractions are specific to help fight the disease causing agents and can be utilized to mount the response of the immune system of the recipient. The positive response is felt after a few hours and especially the next day.
3. In fresh serum are found various necessary or essential molecules such as functional proteins, metabolic fractions, minerals, essential amino acids, trace elements, enzymes that are present and formed only when their activities components are also present in the donor animal and others. This means that the blood used provides the receiver, metabolic fractions to be mounted by the immune system against environmental diseases, and other specific to defend disease present or disease recently cured in the donor animal.
4. The products obtained in this presentation are edible substances and are used in different cultures worldwide as raw meat, undercooked meat, raw liver, raw lung medulla oblongata "simply bulb" raw, crude fat, raw eggs and other parts of the body animals where blood is present in them. Additionally, whole blood, red blood cells, gamma globulin, and other fractions thereof are often used in medicine in injectable form, or infusion. Phospholipids are daily consumption substances found in all living beings, including plants. They are released on world markets for use as natural emulsifiers and stabilizers in foods; in medicine in its diluted form and as a carrier for protective for vaccines, drugs, genes and radiological diagnosis.
5. It is known that active biological substances work orally, such as in the following cases: breast milk for newborns, transmitting maternal antibodies in the milk for babies; the scandal published on the Mac Donald network in the USA to sell sandwiches with undercooked meat, causing symptoms of hyperthyroidism because of the presence of undercooked thyroid gland in the meat; diets combined with protein prescribed to strengthen the patient's body; scientifically guaranteed effects isolated of biological natural substances isolated of animals, plants or human beings as diverse hormonal types, enzymes, vitamins, protein solutions to feed orally, enteral or parenteral weak patients.
6. Providing biological substances useful and essential to the body, strengthen the patients and help the immune system function.
7. The immune system is activated through pathological induction and directed with quantitative and qualitative regulations, where a type of the body's immune system attacks preferably the most dangerous pathological factor in the first stage, ignoring less serious pathological factors to be eliminated and utilized in metabolism itself, or corrected by the defense after it reaches a favorable condition. This grants to treatment with fractionated blood greater importance. We know there are spontaneous healing of cancer, demonstrating the ability of the immune system to manufacture specific antibodies "found in the serum of humans and animals" to combat it and can be used by the treated receiver.
8. The products of high nutritional values help in the treatment of various cancers. The nutritious substances benefit the vital activities of the body, because of the presence of appropriate natural circulations that lack in cancerous mass. This latter is in a state of rapid multiplication leaving the more sensitive to the effects of conventional anticancer treatment and to the attack of specific antibodies which have the ability to penetrate into pathological tissues.
9. The described substances improve states of malnutrition, general weakness and lack of certain nutrients in the meals. They help to complement, with less effort, all biological activities needed in the body because they are vital substances of complete vital circles in living beings.
10. The aqueous solution, gel-like or dry serum of phospholipids and serum once ready for use may be used by healthy people, vegetarian or not, from five (5) months to advanced age (unlimited), pregnant women, breastfeeding women, sick patients to strengthen the body and normalize pathological effects and laboratory values.
11. Counter-indications to the use of blood fractions; allergy to the component of the formula; cases by which to enhance the immune response, would be undesirable such as in the case of transplants and in the presence of Rh negative in pregnancy; in severe renal impairment would be recommended to evaluate the amount of protein in meals, with the applied dosage, in the restriction of protein.

### Summary:

The products offered are thus combined, physically, from two nutritional factors.
A - The blood and its fractions combined with cell membrane phospholipids or their natural or synthetic fractions such as phosphatidylcholine of any origin, are excellent natural food factors that help the vital functions in the prevention of diseases in the body, in recoveries of patients and help in the treatment of cancer and other diseases that can be recognized or registered by the immune system as the antigen;
B - Counter indications are evaluated as in conventional medicine;
C - Our new theoretical part may be correct or not, but it is in accordance to the final results in practice, example: the positive effect on thalassemia.

The following excerpt of this report refers specifically to cancer, the most recent studies on the treatment of this disease and the possible association of this advantageous treatment with the formulation and products of the present invention.

Thus, we have that, currently, there are studies to create cancer vaccines using different types of mono or multinucleated macrophages, vaccines of antigens of cancerous cellular membrane, dendritic cells vaccine, DNA vaccine, and vaccines of tumor cells. These methods encounter certain difficulties that are not to be discussed in this presentation. Source: saude.hsw.uol.com.br/vacina2.htm.

This method consists in taking one or more mammal, vaccinate or implant whole cancer cells taken from the patient's cancer that must be treated. The immune response of the vaccinated animal is the formation of specific antibodies to the tumor, curing the implanted cancer. The antibodies formed by the vaccinated animal can be specific for the implanted cells or their specific markers of their membrane. Therefore, these two types of antibodies formed in the animal are useful to combat implanted cancer cells or their markers on the membranes, because the two forms of activities of the formed antibodies can kill and eliminate the implanted cancer cells. The blood of the vaccinated animal is immunized and rich in specific antibodies against the implanted cancer and, in addition, valued as a nutritious product, providing fragments half ready to patients to assemble their specific antibodies against cancer in a more powerful and faster way, when taken orally. The product also benefits other patients with the same type of cancer.

The overall effect of the product is similar to the effect of blood serum taken orally to increase the overall capacity of the immune system in the recipient be it carrier of the same type of cancer, another type of cancer or person without cancer disease as provided for in the present invention.

Procedure: The process of obtaining the product or prevention action against cancer according to the invention comprises therefore the steps of:
1 - Inoculate in a mammal infected with the target cancer cells taken from a human patient.
2 - Between the fifth and seventh day perform a single withdrawal of blood from the animal inoculated within the allowable limit, taking into account the survival of the animal.
3 - Separate blood serum taken from the inoculated animal or its gamma globulins extracted.
4 - In each amount of 1 liter of separated serum or its extracted gamma globulin mix phospholipids of the cell membrane or their natural or synthetic fractions preferably phosphatidylcholine at a ratio of 0 to 500 grams.
5 - Dilute in 1 to 1.5 liter of water or other diluent and split into portions 20 to 30 ml; and
6 - Freeze, following the same criteria of validity of other products for oral use.

Formulation: Therefore the formulation obtained is comprised of: For blood serum taken from inoculated mammal with a target cancer or gamma globulins extracted from blood inoculated at a ratio of 1L; by cell membrane phospholipids or their natural or synthetic phosphatidylcholine fractions preferably at a ratio of 0 to 500 grams; by 1 to 1.5 liters of water or other diluent.

In this case, the recommended dose is a portion of 20 to 30 ml 1 to 3 times a day orally, according to the needs of each patient.

This formulation also works to obtain products according to the present invention.

Justification: We know we cannot activate a type of immune system against two or more kinds of antigens at the same time, thus the method of producing antibodies in mammals and taken as a nutrient does not confuse the immune system of the recipient and will have no effect side.

Counter indications: allergy to the product's component; calculate the amount of protein in meals in the case of severe renal insufficiency.

The use of serum taken orally is counter indicated in cases in which to strengthen the immune response would be undesirable, such as in the case of transplants and in the presence of Rh negative in pregnancy.

## Claims

1. Formulation based on food factors **characterized by** dietary factors comprised from blood or derived from blood fractions combined with phospholipids.

2. Formulation, according to claim 1, **characterized by** phospholipids or their natural or synthetic fractions.

3. Formulation, according to claim 1 or 2, **characterized by** comprising for each 1 liter of blood derivative or of derivative of blood fractions 0 to 500 grams of phospholipids or phospholipids of cell membrane or its natural or synthetic fractions, for oral use and 1 to 5 grams for injectable use, respectively.

4. Formulation, according to any one of claims 1, 2, 3, **characterized in that** the blood derivative or the blood fractions comprises: whole blood; blood serum; red blood cells or hemoglobin; gamma globulin isolated from the serum of homologous or heterologous origin of mammals; specific gamma globulin, of type serum or antiserum for bites of poisonous animals; whole blood or red blood cells isolated from human blood.

5. Formulation, according to claim 2, **characterized in that** it comprises phospholipids or their natural or synthetic fractions in aqueous solutions, blood serum or saline solution.

6. Product based on food factors, obtained with the use of the formulation of claims 1 to 5, **characterized in that** it comprises additionally an biological stabilizer to be combined with isolated type of gamma globulin of blood serum of homologous origin; said biological stabilizer comprising phospholipids or their natural or synthetic fractions in saline solution or not.

7. Product, according to claim 6, **characterized in that** it comprises for each 1 liter of gamma globulin isolated from the blood serum of homologous origin 1 to 5 grams of biological stabilizer comprising phospholipids or its natural or synthetic fractions.

8. Product, according to claim 6 and 7, **characterized by** being injectable

9. Product based on food factors obtained with the use of the formulation of claims 1 to 5, **characterized in that** it comprises, additionally, isolated gamma globulin isolated from blood serum of homologous origin, combined with biological stabilizer comprising phospholipids or their natural or synthetic fractions in aqueous solution or other diluents or not.

10. Product, according to claim 9, **characterized in that** it comprises for each 1 liter of gamma globulin 0 to 500 grams of biological stabilizer such as phospholipids or their natural or synthetic fractions.

11. Product, according to claims 9 and 10, **characterized by** being orally administered.

12. Product based on food factors, obtained with the use of the formulation of claims 1 to 5, **characterized by** additionally comprising gamma globulin isolated from blood serum, of mammal species, combined or not with biological stabilizer comprising phospholipids or their natural or synthetic fractions in aqueous solution or other diluents or not and pasteurized.

13. Product, according to claim 12, **characterized in that** it comprises for each 1 liter of gamma globulin 0 to 500 grams of biological stabilizer such as phospholipids or their natural or synthetic fractions.

14. Product, according to claims 12 and 13, **characterized by** being orally administered.

15. Product based on food factors obtained with the use of the formulation of claims 1 to 5, **characterized in** antiserum, further comprising specific gamma globulin against bites of poisonous animals, combined or not with biological stabilizer such as phospholipids or their natural or synthetic fractions in aqueous solution or other diluents or not, and pasteurized.

16. Product, according to claim 15, **characterized in that** it comprises for each 1 liter of specific gamma globulin against bites of poisonous animals 0 to 500 grams of biological stabilizer such as phospholipids or their natural or synthetic fractions.

17. Product, according to claims 15 and 16, **characterized by** ability to be orally administered.

18. Product based on food factors, obtained with the use of the formulation of claims 1 to 5, **characterized in that** it comprises, additionally, serum extracted from blood of mammalian species, combined with biological stabilizer comprising phospholipids or their natural or synthetic fractions dissolved in aqueous solution or in other diluents or not, and pasteurized.

19. Product, according to claim 18, **characterized in that** it comprises for each 1 liter of serum extracted from the blood, of species of mammals 0 to 500 grams of biological stabilizer such as phospholipids or its fractions or natural synthetic.

20. Product, according to claims 18 and 19, **characterized by** ability to be orally administered.

21. Product based on food factors, obtained with the use of the formulation of claims 1 to 5, **characterized in that** it comprises, in addition, the combination of phospholipid solution or its natural or synthetic fractions; blood serum of mammals; and natural juices.

22. - Product, according to claim 21, **characterized in that** it comprises for each 1 liter of blood serum of mammals 0 to 500 grams of phospholipid solution or its natural or synthetic fractions; and natural juices.

23. Product, according to claims 21 and 22, **characterized by** ability to be orally or enterally administered.

24. Product based on food factors obtained with the use of the formulation of claims 1 to 5, **characterized in that** it further comprises red blood cells extracted from blood of mammals, hydrolyzed, diluted or not, and combined with biological stabilizer such as phospholipids or their natural or synthetic fractions, in aqueous solution or other diluents or not, pasteurized solution.

25. Product, according to claim 24, **characterized in that** it comprises for each 1 liter of red blood cells taken from the blood of mammals 0 to 500 grams of biological stabilizer such as phospholipids or their natural or synthetic fractions; and 0.5 to 1 liters of aqueous solution or other diluents or not, pasteurized.

26. Product, according to claims 24 and 25, **characterized by** being orally administered.

27. Product based on food factors obtained with the use of the formulation of claims 1 to 5, **characterized by** comprising, additionally, blood derivatives or of its fractions of homologous or heterologous origin of species of hot blood combined with biological stabilizer comprising phospholipids or their natural or synthetic fractions in aqueous solution or other diluents or not.

28. Product, according to claim 27, **characterized in that** it comprises for each 1 liter of blood product or its fractions of homologous or heterologous origin of hot blooded species from 0 to 500 grams of biological stabilizer comprising phospholipids or their natural or synthetic fractions in aqueous solution or other diluents or not.

29. Product, according to claims 27 and 28, **characterized by** being orally administered.

30. Product based on food factors obtained with the use of the formulation of claims 1 to 5, **characterized in that** it comprises, additionally, blood derivative or animal blood fractions, effective against a targeted disease, disorder, of humans combined with biological stabilizer comprising phospholipids or their natural or synthetic fractions, dissolved in aqueous solution or other diluents or not.

31. Product, according to claim 30, **characterized in that** it comprises for each 1 liter of blood derivative or blood fractions of animals, effective against a targeted disorder, diseases, of humans 0 to 500 grams of biological stabilizer comprising phospholipids or their natural or synthetic fractions; and 0.5 to 1 liters of aqueous solution or other diluents or not.

32. Product, according to claims 30 and 31, **characterized by** doses originally frozen of oral administration.

33. Product based on food factors obtained with the use of the formulation of claims 1 to 5, **characterized in that** it additionally comprises blood of mammals, hydrolyzed or not with biological stabilizer such as phospholipids or their natural or synthetic fractions in aqueous solution or other diluents or not.

34. Product, according to claim 33, **characterized in that** it comprises for each 0.5 to 1 liters of blood of mammal animal, hydrolyzed or not 0 to 500 grams of biological stabilizer such as phospholipids or their natural or synthetic fractions, in aqueous solution, or other diluents or not.

35. Product, according to claims 33 and 34, **characterized by** ability to be orally administered.

36. Product based on food factors, obtained with the use of the formulation of claims 1 to 5, **characterized in that** it further comprises whole blood or solution of red blood cells isolated from human blood, combined with biological stabilizer comprised of phospholipids or their natural or synthetic fractions, diluted with saline.

37. Product, according to claim 36, **characterized in that** whole blood or solution of red blood cells isolated from human blood in the ratio of 1 liter ; and by biological stabilizer comprised of phospholipids or their natural or synthetic fractions, diluted with saline at a ratio of 1 to 5 grams, dissolved in 2 to 10 ml of saline.

38. Product, according to claims 36 and 37, **characterized by** ability to be administered by infusion.

39. Product based on food factors obtained with the use of the formulation of claims 1 to 5, **characterized in that** it additionally comprises serum or its gamma globulin isolated from blood of mammal animals, previously vaccinated against the disease target, combined with a biological stabilizer comprising phospholipids or their natural or synthetic fractions in aqueous solution or other diluents or not, pasteurized or not.

40. Product, according to claim 39, **characterized by** serum or its gamma globulin isolated from blood of mammal animals previously vaccinated against the target disease.

41. Product, according to claims 39 and 40, **characterized by** serum or its gamma globulin isolated from the blood of the mammal animals previously vaccinated against target disease in the ratio of 1 liter; mixed with 0 to 500 grams of biological stabilizer such as phospholipids or their natural or synthetic fractions; and aqueous solution or other diluents in the proportion of 0.5 to 1 liters.

42. Product, according to any one of claims 39 to 41, **characterized by** ability to be orally administered.

43. Product based on food factors, according to any one of claims 6 to 42, **characterized in that** it comprises adjuvants pharmaceutically and/or nutritionally suitable.

44. Product, according to claim 43, **characterized by** adjuvants pharmaceutically and/or nutritionally suitable comprising: combined lecithin phosphatidylserine, phosphatidylethanolamine, phosphatidylcholine, phosphatidylinositol, sphingomyelin and other non-toxic fractions generally understood as phosphatidylcholine; lecithin, phosphatidylcholine, phosphatidylinositol, phosphatidylethanolamine and its other fractions extracted from soy, eggs, cereal and the like; whole blood, erythrocytes, gamma globulin.

45. Method for obtaining of the formulation of claims 1 to 5, comprising:
- Preparing of blood derivatives or blood fractions (gamma globulin, serum, red blood cells, hemoglobin or blood) at low temperature, standard in the handling of fresh or raw biological substances, 3 up to 10° C;
- Stabilizing blood derivatives or blood fractions (gamma globulin, hemoglobin, serum, red blood cells or blood), prepared in low temperature, using phospholipids or their natural or synthetic fractions in the proportion 0 to 500 grams in one liter of solution fractionated of blood used orally; or 1 to 5g per liter for infusion used intra-arterial intravenously;
- Dissolving blood derivatives or of blood fractions (gamma globulin, hemoglobin, serum, red blood cells or blood) stabilized with phospholipids or their natural or synthetic fractions in water or saline solution in equal volumes or not;
- Optionally, pasteurizing the formulation obtained;
- Dividing into portions (doses) comprising at least 10 ml of blood fraction; and
- Freezing.

46. Method for obtaining, according to claim 45, wherein the phospholipids may be sterilized before being mixed with other components.

47. Method for obtaining, according to claims 45 and 46, **characterized by** blood fractions and phospholipids or its natural or synthetic fractions comprised in any suitable concentration.

48. Method for obtaining, product of claims 6 to 44, **characterized by** the use of the formulation of claims 1 to 5 and the use of adjuvants pharmaceutically and / or nutritionally suitable selected from among: lecithin combined phosphatidylserine, phosphatidylethanolamine, phosphatidylinositol, phosphatidylcholine, sphingomyelin and other non-toxic fractions generally understood as phosphatidylcholine; lecithin, phosphatidylcholine, phosphatidylinositol, phosphatidylethanolamine and other soybean fractions extracted from their eggs, cereals and the like; whole blood, erythrocytes, gamma globulin.

49. Method for obtaining according to any one of claims 45 to 48, **characterized by** lyophilization of the formulation or product.

50. Method for obtaining, according to any one of claims 45 to 48, **characterized by** encapsulation of the formulation or product.

51. Method for obtaining according to any one of claims 45 to 48 **characterized by** aqueous solution of blood fractions conserved in gelatin, soft or hard, divided into portions.

52. Formulation based on food factors, comprising for each 1liter of blood serum taken from mammal inoculated with a target cancer or by gamma globulins extracted from inoculated blood 0 to 500 grams of phospholipids or its natural or synthetic fractions; and 1 to 1.5 liters of water or other diluent.

53. Method for obtaining formulation of claim 52, comprising:
1 - Inoculating infected cells in a mammal with the target cancer taken from a human patient;
2 - Between the fifth and seventh day perform a single withdrawal of blood inoculated animal within the allowable limit, taking into account the survival of the animal;
3 - Separating the serum of the drawn blood from the animal inoculated or its extracted gamma globulins;
4 - In each amount of 1 liter of serum separated or its gamma globulin extracted, mix phospholipids or their natural or synthetic fractions, in proportion of 0 to 500 grams;
5 - Diluting in 1 to 1.5 liters of water or other diluent and split into portions 20 to 30 ml; and
6 - Freezing, following the same criteria of validity of other products of oral use.

## Amended claims

### Amended claims under Art. 19.1 PCT

The following claim listing replaces all previous claim listings.
**1.** 53 (Canceled)
**54.** New) A natural formulation to improve the health of a patient comprising:
dietary factors contained in blood fractions, and
biological stabilizers.
**55.** The natural formulation as defined in claim 54, further comprising:
said dietary factures are immune system components.
**56.** The natural formulation as defined in claim 55, further comprising:
said immune system components are derived from gamma globulin isolated from the serum of mammals.
**57.** The natural formulation as defined in claim 56, further comprising:
said mammals are of homologous origin.
**58.** The natural formulation as defined in claim 54, further comprising:
said mammals are of heterologous origin.
**59.** The natural formulation as defined in claim 54, further comprising:
said biological stabilizer is a phospholipid.
**60.** The natural formulation as defined in claim 59, further comprising:
said phospholipid is phosphatidylcholine.
**61.** The natural formulation as defined in claim 54, further comprising:
said formulation is injected into the patient.
**62.** The natural formulation as defined in claim 54, further comprising:
said formulation is taken orally by the patient.
**63.** The natural formulation as defined in claim 54, further comprising:
said formulation is stored, prior to administration to the patient by a method selected from the group consisting of: aqueous solution, lyophilized to be dissolved in aqueous solution just prior to use, frozen to be administered immediately after thawing,
**64.** The natural formulation as defined in claim 54, further comprising natural juices.
**65.** The natural formulation as defined in claim 54, further comprising:
said dietary factors further comprise serum or antiserum for bites of poisonous animals.
**66.** The natural formulation as defined in claim 54, further comprising:
said dietary factors further comprise blood fractions effective against a targeted disease or a disorder of humans.
**67.** A method of preparation of a natural formulation comprising:
obtain fresh or raw biological substances,
cool the substances in a range from 3° C to 10° C,
isolating whole blood from said biological substances and isolating blood derivatives selected from: gamma globulin, serum, red blood cells and hemoglobin
adding a biological stabilizer,
dissolving blood derivatives in solutions selected from: water and a saline solution.
**68.** A method of preparation as defined in claim 67 further comprising:
said biological stabilizer is a phospholipid.
**69.** A method of preparation as defined in claim 67 further comprising:
said preparation is prepared for storage using methods selected from the group consisting of: pasteurization, freezing, lyophilization.
**70.** A method of preparation as defined in claim 67 further comprising:
wherein said fresh or raw biological substances are conserved in hard gelatin or soft gelatin.
**71.** A method of preparation as defined in claim 67 further comprising:
said blood derivatives are derived from a mammal previously vaccinated against a target disease.
**72.** A method of preparation as defined in claim 67 further comprising:
said blood derivatives are derived from a mammal inoculated with cancer.
**73.** A method of preparation as defined in claim 67 further comprising adjuvants selected from the group consisting of: lecithin combined phosphatidylserine, phosphatidylethanolamine, phosphatidylinositol, phosphatidylcholine, sphingomyelin and lecithin.
